# EUROPEAN PATENT SPECIFICATION

(11) **EP 0 432 263 B1**
(45) Date of publication and mention of the grant of the patent: **09.03.1994**
(21) Application number: 88907767.3
(22) Date of filing: 05.09.1988
(51) Int. Cl.: A61F 13/20

(54) **SANITARY TAMPON**
DAMENBINDE
TAMPON HYGIENIQUE

(43) Date of publication of application: 19.06.1991
(73) Proprietor: SHIMATANI, Sumie, Kouza-gun, Kanagawa 253-01 (JP)
(72) Inventor: SHIMATANI,Kazuo, Kouza-gun Kanagawa 253-01 (JP)
(74) Representative: Hillier, Peter
(86) International application number: JP8800887
(87) International publication number: WO9002541

(56) References cited:
- WO-A-89/06524
- WO-A-89/07924
- WO-A-89/07925
- GB-A- 537 113
- JP-B- 6 114 826
- JP-U- 6 148 724

## Description

### FIELD OF THE INVENTION

The present invention relates to a sanitary tampon which is highly absorptive for menstrual discharge, absorbs menstrual discharge without allowing it to leak out, and is easy to take out of the body after use.

### DESCRIPTION OF THE PRIOR ART

Tampons of various constructions are used for menstruation and other such instances, and are classified roughly into two types. One of them is prepared by forming an absorbent material such as absorbent cotton, absorbent paper or the like into a broad band, winding the band tightly into a roll, and applying pressure to the roll from outer circumferential directions to form a bullet-like product. The other type is prepared by compressing an absorbent material in band form vertically and horizontally to obtain a stick-like product.

The known tampons shaped through compression processes as noted above have various appurtenant constructions to be inserted with ease into the vagina. However, these tampons basically have the following problems.

The former type tampon absorbs menstrual blood and swells radially in the vagina. This tampon, although it reliably absorbs blood flowing down vaginal walls, is not easy to pull out of the body after use since it is swollen and has an increased diameter. It is difficult for the tampon to contract when recovered from the body, and a pull string attached to the tampon is broken often so that the user must see a doctor to have the tampon removed from her.

As regards the latter type tampon, it swells vertically after absorbing menstrual blood and hence has the advantage over the former type of easy recovery from the body. However, this tampon has the problem of gaps being formed between the tampon and vaginal walls during use, whereby blood is not absorbed in a satisfactory manner.

GB-A-537113 discloses a sanitary tampon manufactured by forming a main tampon body from a blood absorbing material having high water absorbing characteristics such as absorbant cotton, and compressing the tampon body into a bullet-like shape.

### SUMMARY OF THE INVENTION

An object of the present invention is to provide a sanitary tampon which effectively absorbs menstrual blood regardless of the quantity thereof and is easy to recover from the body.

According to one aspect of the present invention, there is provided a sanitary tampon manufactured by forming a main tampon body from a blood absorbing material having high water absorption characteristics such as absorbent cotton, and compressing the main tampon body into a bullet-like shape characterised by, during manufacture, forming the blood absorbing material into a string, knitting tampon material with the string, and forming the main tampon body from the tampon material.

The tampon according to the present invention has a large surface area and includes meshes defining a multiplicity of voids, to absorb menstrual blood efficiently during use. Each line of the string having absorbed menstrual blood is rapidly swollen independently of the other, whereby the tampon returns substantially to a pre-compression state to positively absorb blood flowing down vaginal walls.

This tampon may be taken out of the body with ease since the tampon is reduced in size and in surface area with the meshes collapsing through contact with vaginal walls at the external opening of the vagina.

Consequently, the present invention allows a person having a small vagina diameter to use a bulky tampon having more than twice the volume, namely more than twice the absorption capacity of a conventional product, for example. In practice, such a bulky tampon is capable of positively absorbing blood influx with more than twice the efficiency.

According to another aspect of the present invention, there is provided a method of manufacturing a sanitary tampon comprising the steps of forming blood absorbing material having high water absorption characteristics, such as absorbent cotton, into a main tampon body and compressing the main tampon body into a bullet-like shape, characterised in that the forming of the main tampon body is carried out by forming the blood absorbing material into a string, knitting tampon material with the string, and forming the main tampon body from the tampon material.

### BRIEF DESCRIPTION OF THE DRAWINGS

The drawings illustrate sanitary tampons embodying the present invention, in which:-
Fig. 1 is a view illustrating a string for forming a tampon material according to a first embodiment of the present invention,
Fig. 2 is a plan view of the tampon material,
Fig. 3 is a perspective view of a cylindrical body formed with the tampon material,
Fig. 4 is an end view of the cylindrical body for illustrating pleats formed thereon,
Fig. 5 is a view showing an outward appearance of a tampon according to the first embodiment,
Figs. 6 and 7 are perspective views of a second embodiment of the invention, respectively,
Figs. 8 and 9 are side views of a third embodiment of the invention, respectively,
Figs. 10 and 11 are perspective views of a fourth embodiment of the invention, respectively, and
Figs. 12 and 13 are perspective views of a fifth embodiment of the invention, respectively.

### DETAILED DESCRIPTION OF THE PREFERRED EMBODIMENTS

Several embodiments of the present invention will be described in detail hereinafter with reference to the drawings.

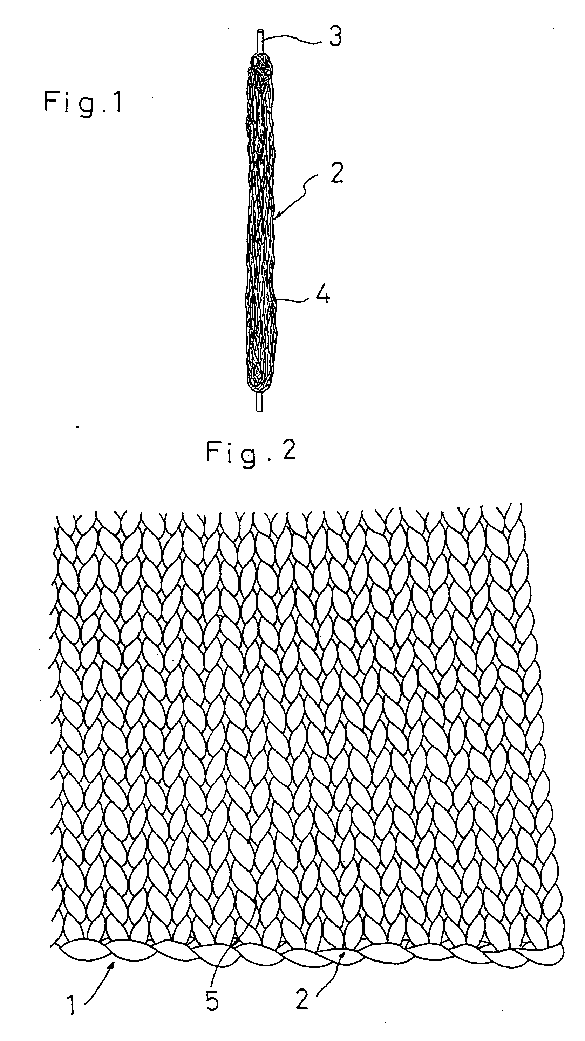

Referring to Figs. 1 through 5 showing a first embodiment of the invention, a tampon material 1 (Fig. 2) according to this embodiment is knitted with a string 2 shown in Fig. 1. The string 2 is formed to have a thickness of 5-8mm, for example, by a single line or plural lines of fiber yarn 3 entwined with single fiber 4 which comprises absorbent cotton, chemical fiber or the like having high water absorption characteristics and suited for use as a tampon component.

The tampon material 1 is loose-knit with the string 2 to have a surface area according to the size of a finished tampon, i.e. S-size, M-size or L-size. The method of intertwining the string 2 is not limited to a particular method, but whatever method is employed it is desirable to knit the tampon material 1 loosely to define meshes 5. The illustrated example employs what is known as stockinette stitch.

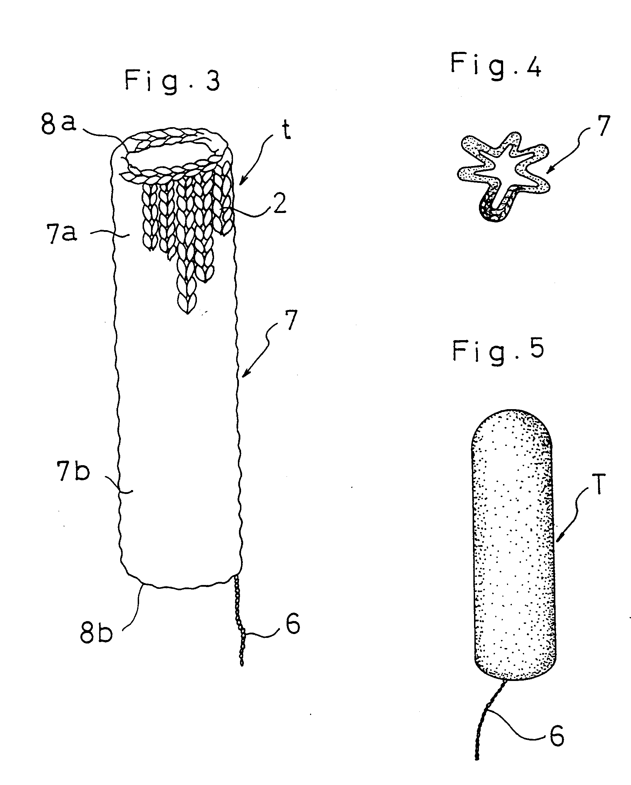

Reference number 6 in the drawings indicates a pull string formed by drawing part of the string 1 from the lower end of the tampon material 1 (see Figs. 3 and 5).

Opposite lateral edges of the tampon material 1 are brought into abutment and stitched together to form a cylindrical body 7 acting as a main body of the tampon t as shown in Fig. 3. This cylindrical body 7 may be stitched up at the top to become a pouch-like body. Further, the cylindrical body 7 may be knitted directly into a tube shape with the string 2.

The cylindrical body 7 thus formed is subjected to primary compression, whereby the cylindrical body 7 is deformed to have a star-shaped section, as shown in Fig. 4, throughout from the upper half 7a to the lower half 7b, i.e. to have peripheral pleats extending over its entire length. Thereafter the cylindrical body 7 is compressed vertically and radially into a bullet-like tampon T as shown in Fig. 5.

This tampon T may be inserted into the vagina with fingers or by means of an applicator, not shown, as is a conventional product. Since the tampon material 1 is knitted with the string 2 comprising a blood absorptive material, its surface area will enlarge to a great extent with a multiplicity of voids defined by meshes 5, whereby menstrual blood is absorbed efficiently and rapidly.

The tampon T having absorbed blood is swollen and expands radially and vertically to return to the shape of cylindrical body 7 promptly as shown in Fig. 3. Since the tampon material 1 is not a continuously planar material but is knitted with the string 2 having high water absorption characteristics as described, each line of the string 2 tends for restoration of its original shape independently of the other, thereby giving full play to its restoring force. Consequently, the tampon T returns substantially to the pre-compression state shown in Fig. 3.

The tampon T may easily be taken out of the body after use by pulling the pull string 6. Though the vagina is constricted adjacent its external opening as distinct from the depth thereof where the tampon T is placed, the tampon T being pulled out is compressed by narrowing vaginal walls adjacent the external opening. This compression is possible because the tampon T after absorbing blood has a shape similar to the cylindrical body 7 which is hollow along its axis and also defines voids at the meshes 5.

At the time of removal, the part of the tampon T inside the vagina has outer surfaces of thick mesh formed by the string 2 contacting vaginal walls. This construction, unlike the simple planar surface contacting the vaginal walls, has the effect of sweeping stains off the vaginal walls as the tampon T is taken out.

Furthermore, since the pull string 6 is linked to the intricacy of the string 2 constituting the tampon material 1, the pulling force acts through the pull string 6 upon the entire tampon material 1. Thus there is no possibility of the pull string 6 breaking at an intermediate position.

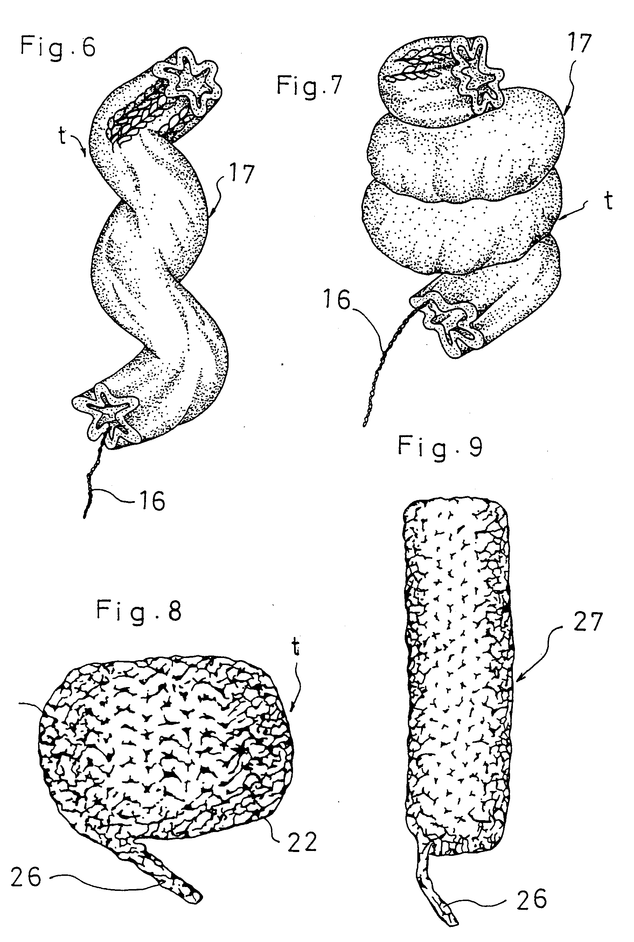

Figs. 6 and 7 show a second embodiment of the present invention, in which the cylindrical body 7 or main body t having the vertical pleats after the primary compression, as shown in Fig. 4, is twisted and coiled as referenced 17 which is then subjected to final compression.

According to this embodiment, when a pull string 16 is pulled after use, the twisted main body t is gradually straightened out as shown in Fig. 6, whereby the tampon is taken out of the body even more easily.

Figs. 8 and 9 show a third embodiment of the invention, in which the tampon material 1 is knitted with a string 22 in such a way that the main tampon body t in a soft ball-like form, when taken out of the body, becomes elongated in the takeout direction.

Thus the main tampon body t having absorbed blood in the vagina returns to the shape shown in Fig. 8. When a pull string 26 is pulled, the main body t is deformed into an elongate body 27 as seen in Fig. 9 to facilitate its recovery from the body.

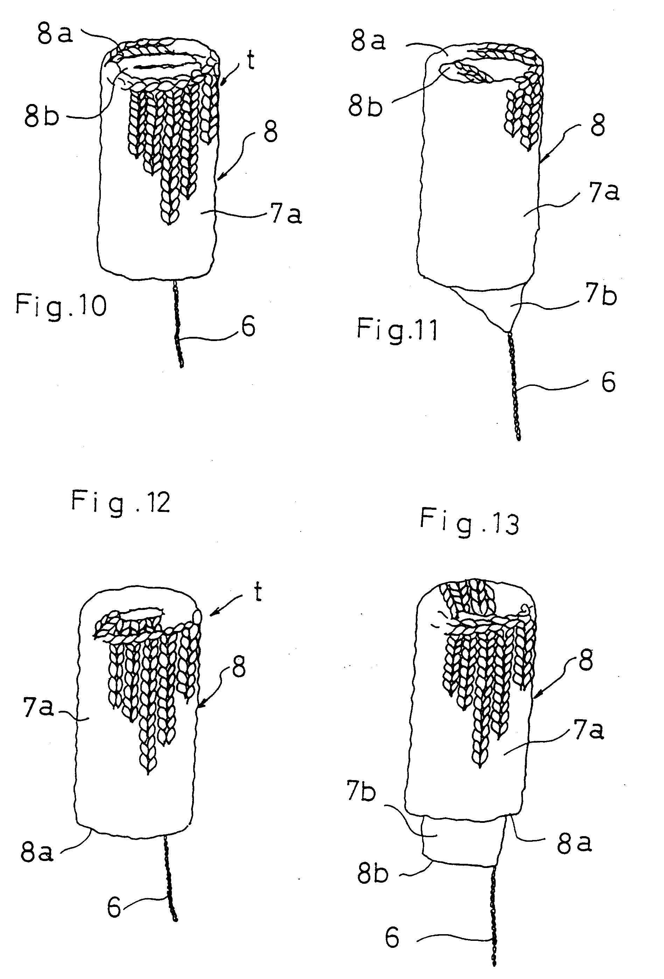

Figs. 10 and 11 show a fourth embodiment of the invention, in which the main tampon body t formed cylindrical as shown in Fig. 3 is changed into a double tube body 8 with the length reduced to half and the diameter increased slightly. This double tube body 8 is formed by inwardly folding, at at an intermediate position longitudinally of the main body t, the lower half 7b having the pull string 6 and gradually advancing the fold upward until the lower end 8b is substantially flush with the upper end 8a.

When the pull string 6 is pulled for taking out the used tampon, the inner tube is gradually pulled down led by the upper end 8b thereof as shown in Fig. 11 and the main body t finally becomes a single tube construction as shown in Fig. 3.

Figs. 12 and 13 show a fifth embodiment of the invention, in which a double tube body 8 with the length reduced to half is obtained in the opposite way to the fourth embodiment. That is, the upper half 7a of the cylindrical body 7 in Fig. 3 is folded over the lower half 7b. Consequently, as distinct from the fourth embodiment, the pull string 6 is connected to the lower end of the double tube body 8. When the pull string 6 is pulled for recovery of the tampon, the inner tube 7b is gradually pulled down as shown in Fig. 13 whereby the double tube body 8 changes into a single tube construction.

These double tube bodies 8 are compressed radially and, when desired, vertically to shape each of them into the bullet-like tampon T as shown in Fig. 5.

The tampon T having absorbed menstrual blood during use returns to the state as shown in Fig. 10 or 12. Although the double tube body 8 is slightly swollen with the blood, it is hollow along its axis and also defines voids at the meshes 5. When passing through the constricted part of the vagina adjacent the external opening after leaving the broader depth thereof, the cylindrical body 7 as now pulled outward in a double tube construction is pressed in by the narrowing vaginal walls adjacent the external opening. The tampon T is thus taken out of the body with ease. The part of the tampon T in the vaginal as the tampon T is being pulled outwardly has outer surfaces of thick mesh formed by the string 2 in contact with the vaginal walls. These outer surfaces leave the vagina, sweeping stains off the vaginal walls.

The string 2 used for knitting the tampon material 1 may comprise cotton or the like just formed into a somewhat thick string, instead of including fiber yarn as the core as in the foregoing embodiments.

Further, the tampon material 1 knitted with the string 2 may simply be rolled into a bar form as in the prior art, and thereafter compressed radially and, when desired, vertically to form the tampon T. The tampon T thus formed, of course, provides the same advantages as the described embodiments.

## Claims

1. A sanitary tampon manufactured by forming a main tampon body (7) from a blood absorbing material having high water absorption characteristics such as absorbent cotton, and compressing the main tampon body (7) into a bullet-like shape characterised by, during manufacture, forming the blood absorbing material (1) into a string (2), knitting tampon material (1) with the string (2), and forming the main tampon body (7) from the tampon material (1).

2. A sanitary tampon as claimed in claim 1, wherein part of the string (2) forming the tampon material (1) acts as a pull string (6) for tampon recovery.

3. A sanitary tampon as claimed in claim 1, wherein the tampon material (1) is knitted in a way to have elongation in a direction of tension.

4. A sanitary tampon as claimed in claim 1, wherein the tampon material (1) is joined at opposite lateral edges thereof to form a cylindrical main tampon body (7).

5. A sanitary tampon as claimed in claim 1 or 4, wherein the tampon material (1) is folded into a double tube construction with walls doubled radially thereof.

6. A method of manufacturing a sanitary tampon comprising the steps of forming blood absorbing material having high water absorption characteristics, such as absorbent cotton, into a main tampon body (7) and compressing the main tampon body (7) into a bullet-like shape, characterised in that the forming of the main tampon body (7) is carried out by forming the blood absorbing material into a string (2) , knitting tampon material (1) with the string (2), and forming the main tampon body (7) from the tampon material (1).

7. A method according to claim 6, wherein the tampon material (1) is joined at opposite lateral edges thereof to form a cylindrical main body tampon body (7).

## Patentansprüche

1. Damentampon, der durch Bilden eines Haupttamponkörpers (7) aus einem blutaufsaugenden Material mit hohen Wasseraufsaugeigenschaften, wie Saugwatte, und durch Zusammenpressen des Haupttamponkörpers (7) in eine patronenartige Form hergestellt ist, dadurch **gekennzeichnet,** daß während der Herstellung das blutaufsaugende Material (1) zu einem Faden geformt wird, das Tamponmaterial (1) durch den Faden (2) gestrickt wird und der Haupttamponkörper (7) aus dem Tamponmaterial (1) gebildet wird.

2. Damentampon nach Anspruch 1, dadurch **gekennzeichnet,** daß ein Teil des das Tamponmaterial (1) bildenden Fadens (2) als ein Ausziehfaden (6) zum Tamponausführen dient.

3. Damentampon nach Anspruch 1, dadurch **gekennzeichnet,** daß das Tamponmaterial (1) so gestrickt ist, daß es eine Längung in Zugrichtung aufweist.

4. Damentampon nach Anspruch 1, dadurch **gekennzeichnet,** daß das Tamponmaterial (1) an seinen gegenüberliegenden seitlichen Rändern so verbunden ist, daß es einen zylindrischen Haupttamponkörper (7) bildet.

5. Damentampon nach Anspruch 1 oder 4, dadurch **gekennzeichnet,** daß das Tamponmaterial (1) zu einer Doppelröhrchenausführung mit in radialer Richtung doppelten Wandungen gefaltet ist.

6. Verfahren zum Herstellen eines Damentampons, das folgende Verfahrensschritte aufweist: Formen von blutaufsaugendem Material mit hohen Wasseraufsaugeigenschaften, wie Saugwatte, zu einem Haupttamponkörper (7) und Zusammenpressen des Haupttamponkörpers (7) zu einer patronenartigen Form, dadurch **gekennzeichnet,** daß das Formen des Haupttamponkörpers (7) durch folgende Schritte ausgeführt wird: Formen des blutaufsaugenden Materials zu einem Faden (2), Stricken des Tamponmaterials (1) durch den Faden (2) und Formen des Haupttamponkörpers (7) aus dem Tamponmaterial (1).

7. Verfahren nach Anspruch 6, dadurch **gekennzeichnet,** daß das Tamponmaterial (1) an seinen gegenüberliegenden seitlichen Rändern so verbunden ist, daß es einen zylindrischen Haupttamponkörper (7) bildet.

## Revendications

1. Tampon hygiénique fabriqué en formant un corps principal (7) de tampon à partir d'une matière d'absorption du sang, à caractéristiques élevées d'absorption d'eau, par exemple un coton absorbant, et en comprimant le corps principal de tampon pour le conformer en balle de fusil, caractérisé en ce que la matière absorbant le sang (1) est formée, pendant la fabrication, en une corde (2), la matière (1) de tampon est tricotée avec la corde (2), et le corps principal (7) de tampon est formé à partir de la matière de tampon (1).

2. Tampon hygiénique selon la revendication 1, dans lequel une partie de la corde (2) formant la matière de tampon (1) constitue une corde de traction (6) pour la récupération du tampon.

3. Tampon hygiénique selon la revendication 1, dans lequel la matière de tampon (1) est tricotée de manière à permettre un allongement dans une direction de tension.

4. Tampon hygiénique selon la revendication 1, dans lequel la matière de tampon (1) est assemblée en ses bords latéraux opposés, afin de former un corps principal cylindrique (7) de tampon.

5. Tampon hygiénique selon l'une des revendications 1 à 4, dans lequel la matière de tampon (1) est pliée en une structure à double tube à parois doublées radialement.

6. Procédé de fabrication d'un tampon hygiénique comprenant les étapes consistant à former une matière d'absorption du sang à caractéristiques élevées d'absorption d'eau, par exemple un coton absorbant, en un corps principal (7) de tampon, et à comprimer le corps principal (7) de tampon pour le conformer en balle de fusil, caractérisé en ce que la formation du corps principal (7) de tampon est effectuée en formant la matière absorbant le sang en une corde (2), puis en tricotant la matière de tampon (1) avec la corde (2), et enfin en formant le corps principal (7) de tampon à partir de la matière de tampon (1).

7. Procédé selon la revendication 6, dans lequel la matière de tampon (1) est assemblée en ses bords latéraux opposés, afin de former un corps principal cylindrique (7) de tampon.
